# EUROPÄISCHE PATENTSCHRIFT

(11) **EP 1 529 484 B1**
(45) Veröffentlichungstag und Bekanntmachung des Hinweises auf die Patenterteilung: **03.08.2011**
(21) Anmeldenummer: 04024137.4
(22) Anmeldetag: 09.10.2004
(51) Int. Cl.: A61B 1/12, A61L 2/18

(54) **Verfahren und Vorrichtung zur Analyse von Endoskopkanälen**
Method and apparatus for analysing endoscope channels
Méthode et appareil d'analyse des canaux d'un endoscope

(30) Priorität: 05.11.2003 DE 10352198
(43) Veröffentlichungstag der Anmeldung: 11.05.2005
(73) Patentinhaber: BHT Hygienetechnik GmbH, 86368 Gersthofen (DE)
(72) Erfinder: Annecke, Karl Heinz, Dr., 64625 Bensheim (DE)
(74) Vertreter: von Bülow, Tam

(56) Entgegenhaltungen:
- WO-A-03/077960
- DE-A1- 10 208 035
- US-A- 5 738 824

## Beschreibung

Die Erfindung bezieht sich auf ein Verfahren zur Analyse von Kanälen gemäß dem Oberbegriff des Patentanspruches 1 sowie auf eine Vorrichtung zur Durchführung des Verfahrens gemäß dem Oberbegriff des Patentanspruches 12.

Verfahren und Vorrichtung dieser Art sind aus der DE 102 08 035 A1 bekannt. Zur Überprüfung der Durchgängigkeit von Endoskopkanälen wird dort vorgeschlagen, ein vorbestimmtes Volumen einer Flüssigkeit mit einem vorbestimmten Druck durch den zu überprüfenden Endoskopkanal zu drücken und die Zeitdauer für diesen Vorgang zu messen. Liegt die gemessene Zeitdauer innerhalb vorgegebener Grenzen, so wird der Endoskopkanal als einwandfrei durchgängig bewertet. Ähnliche Verfahren und Vorrichtungen zur Überprüfung langer dünner Kanäle und insbesondere Endoskopkanäle sind auch aus EP 0 711 529 A1, DE 44 34 114 A1, US 5,551,462, DE 36 01 395 A1, EP 0 709 056 A1, US 5,738,824 A, GB 2 275 341 A und EP 0 072 257 A2 bekannt.

Mit diesen bekannten Vorrichtungen und Verfahren läßt sich zwar überprüfen, ob einzelne Kanäle korrekt gereinigt sind und damit vorgegebene Durchlässigkeitskriterien erfüllen oder ob ein Kanal noch einmal gereinigt oder verworfen werden muß.

Es wurde jetzt aber festgestellt, daß lange dünne Kanäle, wie z.B. Endoskopkanäle oder sonstige medizinische Schläuche, ihre Durchlässigkeit im Laufe der Zeit verringern und so nach und nach unbrauchbar werden, selbst wenn die einzelne vorhergehende Reinigung einwandfrei war und die Durchlässigkeit noch innerhalb vorgegebener Grenzwerte lag. Für dieses Phänomen bieten sich verschiedene Erklärungen an:
- das für den Endoskopkanal verwendete Material dehnt sich durch den Kontakt mit Wasser oder anderen Flüssigkeiten aus, womit sich der Kanaldurchmesser kontinuierlich verringert;
- trotz einwandfreier Reinigung bildet sich im Inneren des Endoskopkanals ein Film von Ablagerungen, wie z.B. ein Biofilm aus organischem Material, was zu einer Kanalverengung führt;
- einzelne Kanäle können durch Arbeitswerkzeuge zerkratzt werden, so daß sich bei der eingangs beschriebenen Durchflußprüfung Strömungswirbel bilden, die die Durchlässigkeit verringern. In solchen Kratzern bzw. Furchen können sich Verunreinigungen ablagern.

Eine Möglichkeit zur Lösung dieses Problems besteht darin, ein Endoskop so lange zu benutzen, bis die Durchflußprüfung ggf. auch nach mehrfacher Reinigung zu Meßwerten führt, die außerhalb vorgegebener Grenzwerte liegen, und dann das Endoskop auszutauschen oder zu reparieren. Ein solches Vorgehen führt in der Praxis aber zu den unerwünschten Folgen, daß ein Endoskop zur Unzeit ausfällt, wenn es gerade dringend benötigt wird, oder daß mehrere Endoskope zeitgleich ausfallen und dann ein empfindlicher Engpass entsteht.

Wünschenswert ist es daher, den Trend einer zunehmenden Kanalverengung sicher zu erkennen, um rechtzeitig vor einem Ausfall Wartungs-, Reparatur- oder Austauschmaßnahmen einleiten zu können.

Aufgabe der Erfindung ist es daher, Verfahren und Vorrichtung der eingangs genannten Art dahingehend zu verbessern, daß der Trend zu einer Kanalverengung frühzeitig erkannt wird. Dies soll insbesondere vollautomatisch im Zusammenhang mit dem Reinigungs-/Desinfektionsprozeß ohne zusätzlichen Arbeitsaufwand möglich sein.

Diese Aufgabe wird für das Verfahren durch die im Patentanspruch 1 und für die Vorrichtung durch die im Patentanspruch 12 angegebenen Merkmale gelöst.

Das Grundprinzip der Erfindung liegt darin, daß die Durchlässigkeit für jeden einzelnen Kanal gemessen und gespeichert wird und mit Hilfe einer Trendanalyse der Meßwerte ermittelt wird, ob bzw. wann sich ein Reparatur-, Wartungs- oder Austauschbedarf ergibt. Die Trendanalyse kann mit Hilfe bekannter mathematischer Verfahren durchgeführt werden, beispielsweise durch Mittelwertbildung aller vorliegenden Meßwerte, Bildung eines gleitenden Mittelwertes über jeweils eine vorbestimmte Anzahl zeitlich geordneter zurückliegender Meßwerte usw. Einzelheiten sind in der nachfolgenden Beschreibung angegeben.

Vorzugsweise findet diese Prüfung und Auswertung unmittelbar im Zusammenhang mit der Reinigung des jeweiligen Endoskopkanales statt, und zwar vor, während oder nach der Reinigung.

Zeigt die Trendermittlung an, daß der aktuelle Trendwert außerhalb eines vorgegebenen Bereiches und insbesondere unterhalb eines vorgegebenen Grenzwertes liegt, so wird bereits dann eine Meldung erzeugt, die einen Reparatur-, Wartungs- oder Austauschbedarf anzeigt, selbst wenn der aktuell gemessene Durchflußwert noch innerhalb vorgegebener Grenzen liegt.

Dadurch, daß die Trendermittlung vollautomatisch im unmittelbaren Zusammenhang mit dem Reinigungs-/Desinfektionsprozeß stattfindet, erhält man auch eine wesentliche Zeiteinsparung, die sonst für die bisher übliche manuelle Prüfung der Durchlässigkeit der Kanäle vorgenommen wurde.

Die Durchlässigkeit eines Kanales kann durch eine Durchflußmessung ermittelt werden, beispielsweise indem die Durchflußrate (Volumen pro Zeiteinheit; z.B. in ml/s) gemessen wird oder indem ein vorbekanntes Volumen vollständig durch den Kanal unter vorgegebenem, vorzugsweise konstant gehaltenem Druck durch den Kanal gepreßt wird und die Zeitdauer gemessen wird, innerhalb der das vorbekannte Volumen durch den Kanal geflossen ist. Wenn im folgenden von Meßwert gesprochen wird, so kann dies sowohl die Durchflußrate als auch die Zeitdauer beinhalten.

Im folgenden wird die Erfindung anhand eines Ausführungsbeispieles im Zusammenhang mit der Zeichnung ausführlicher erläutert. Es zeigt:
- Fig. 1: eine Prinzipskizze einer Vorrichtung zur Durchführung des Verfahrens nach der Erfindung;
- Fig. 2: drei Meßreihen der Durchflußrate (in ml/s) über der Anzahl von Messungen (n) anhand von drei Kanälen, die mit reinem Wasser gespült wurden, zur Überprüfung der Systemgenauigkeit;
- Fig. 3: zwei Meßreihen der Durchflußrate unter Verwendung von verschmutztem Wasser;
- Fig. 4: zwei Meßreihen der Durchflußrate mit verschmutztem Wasser unter Zwischenspülung mit reinem Wasser;
- Fig. 5: zwei Meßreihen der Durchflußrate, von denen eine die Messung mit gelockertem Anschluß zeigt;
- Fig. 6: eine Meßreihe einer Durchflußrate und zwei verschiedene Trendlinien von Durchflußraten; und
- Fig. 7: drei Meßreihen von Durchflußraten in vergrößertem Maßstab im Anschluß an die Reinigung von Kanälen.

Es sei darauf hingewiesen, daß die Meßreihen nicht maßstäblich sind und übereinander dargestellte Kurven der Übersichtlichkeit halber gegeneinander verschoben abgebildet sind.

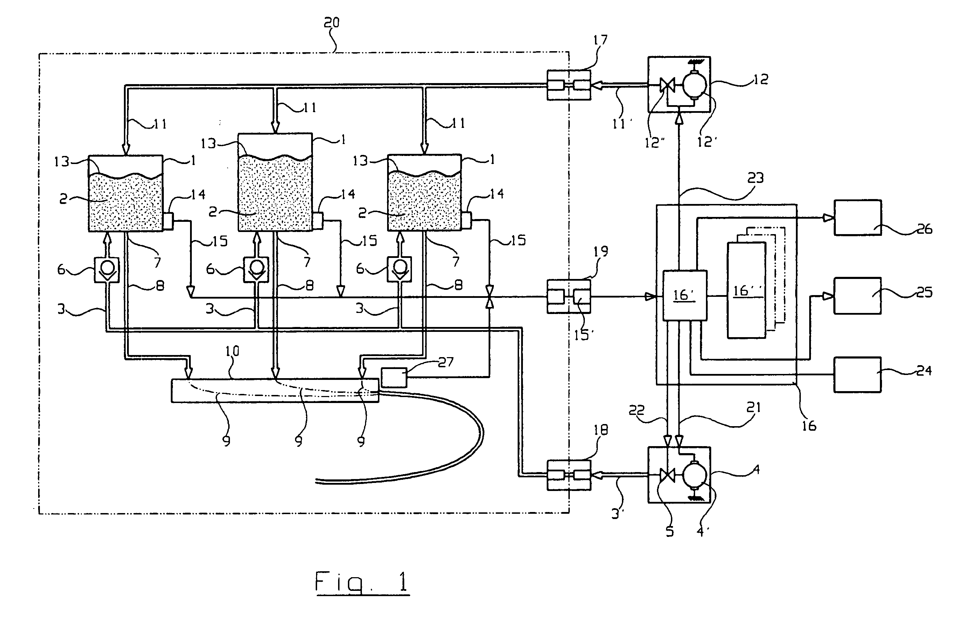

Zunächst wird auf Fig. 1 Bezug genommen. Der grundlegende Aufbau der dort skizzierten Vorrichtung entspricht der Vorrichtung der DE 102 08 035 A1. Sie unterscheidet sich hiervon im wesentlichen nur durch die Ausgestaltung der Steuer- und Auswerteeinheit 16.

Die Vorrichtung hat hier drei Behälter 1 mit einem vorgegebenen Volumen einer Menge einer Flüssigkeit 2. Jeder Behälter 1 ist über eine Leitung 3 mit einer Flüssigkeitsversorgung 4 verbunden, wobei das Befüllen der Behälter 1 über ein steuerbares Ventil 5 erfolgen kann. In die jeweilige Leitung 3 kann auch noch ein Rückschlagventil 6 eingesetzt sein, das verhindert, daß Flüssigkeit aus dem Behälter 1 zurück zur Flüssigkeitsversorgung 4 fließen kann.

Im unteren Bereich jedes Behälters 1 ist ein Auslaß 7 vorgesehen, der über je eine Leitung 8 mit jeweils einem zugeordneten Kanal 9 eines Endoskops 10 verbindbar ist. Weiter sind alle Behälter 1 über eine gemeinsame Leitung 11 mit einer Druckluftquelle 12 verbunden, mittels der die Flüssigkeit 2 in den Behältern 1 mit Druck beaufschlagbar ist. Die Leitung 11 mündet vorzugsweise in den oberen Bereich aller Behälter 1, so daß die Oberfläche 13 der Flüssigkeit 2 mit Druckluft beaufschlagt wird. Im unteren Bereich jedes Behälters 1 ist je ein Sensor 14 angebracht, der über eine Leitung 15 mit einer elektronischen Steuer- und Auswerteeinheit 16 verbunden ist. Diese "Steuereinheit" genannte Einheit 16 überwacht das Ausgangssignal der Sensoren 14 und steuert die Druckluftquelle 12 und die Flüssigkeitsversorgung 4 bzw. das Ventil 5.

Die Sensoren 14 haben allgemein gesprochen die Aufgabe festzustellen, ob der Flüssigkeitspegel der Flüssigkeit 2 unter einem vorgegebenen Wert liegt und insbesondere der jeweilige Behälter 1 vollständig entleert ist. Hierzu können beispielsweise bekannte Füllstandsgeber verwendet werden, wie z.B. ein durch einen Schwimmer betätigbarer Schalter, ein elektronischer oder optischer Sensor, der das Vorhandensein einer Flüssigkeit feststellt. Es kann aber auch ein Drucksensor verwendet werden.

Das Endoskop 10 und alle Behälter 1 sind auf einem gemeinsamen Träger 20 angeordnet, der beispielsweise ein Einsatzwagen sein kann, der in eine nicht dargestellte Reinigungs- und Desinfektionsmaschine eingesetzt werden kann. Der Träger hat hier drei Kupplungen 17, 18, 19, wobei die Kupplung 17 die Druckluftleitungen 11 mit der Druckluftquelle 12 verbindet, die Kupplung 18 die Flüssigkeitszufuhrleitungen 3 mit der Flüssigkeitsversorgung 4 und schließlich die Kupplung 19 die Leitungen 15 und damit die Sensoren 14 mit der Steuereinheit 16 verbindet.

Nach einer Alternative kann auch nur das Endoskop 10 auf dem Träger 20 angeordnet sein, während alle übrigen Komponenten und auch die Behälter 1 außerhalb des Waschraumes in der Maschine angeordnet sein können. Die Kanäle 9 des Endoskops 10 werden über eine Mehrfachkupplung und einem entsprechenden Gegenstück im Waschraum mit den übrigen Komponenten der Maschine verbunden.

Die Steuereinheit 16 ist darüber hinaus über elektrische Leitungen 21 und 22 mit der Flüssigkeitsversorgung verbunden, die hier beispielsweise eine Pumpe 4' und das Absperrventil 5 enthält. Weiter ist die Steuereinheit 16 über eine elektrische Leitung 23 mit der Druckluftquelle 12 verbunden, die beispielsweise einen elektrisch angetriebenen Kompressor 12' und ggf. ein Absperrventil 12" aufweist.

Das Endoskop 10 wird bei der einen Alternative noch außerhalb der Reinigungs- und Desinfektionsmaschine auf dem Träger 20 über die Leitungen 8 mit den einzelnen Behältern 1 verbunden, wobei darauf hinzuweisen ist, daß Endoskope bis zu zehn oder mehr Kanäle 9 aufweisen können, die einzeln anzuschließen sind. Bei der anderen Alternative erfolgt der Anschluß über die genannte Mehrfachkupplung. Bei der ersten Alternative muß der Träger 20 nur über drei Kupplungen 17, 18 und 19 mit Anschlüssen in der Maschine verbunden werden, was entweder von Hand oder auch über automatische Kupplungen erfolgen kann. Die der Maschine zugeordnete Leitungen sind mit 3', 11' und 15' bezeichnet. Weiter ist noch darauf hinzuweisen, daß die einzelnen Behälter 1 unterschiedliche Größen haben können und damit an die Querschnitte der zu prüfenden Kanäle 9 angepaßt werden können.

Die Steuereinheit 16 enthält einen Rechenbaustein, wie z.B. einen Mikrocontroller 16' sowie mindestens einen Speicherbaustein 16", der mit dem Mikrocontroller 16' verbunden ist. Weitere bei Steuerungen von Reinigungs- und Desinfektionsmaschinen verwendete Bauteile, wie Eingabe-/Ausgabe-Schnittstellen, Treiberschaltungen zur Ansteuerung von Ventilen, Elektromotoren etc. sind ebenfalls in der Steuereinheit 16 enthalten, aus Gründen der Übersichtlichkeit jedoch nicht im Detail dargestellt.

An die Steuereinheit 16 bzw. den Mikrocontroller 16' sind noch weitere Peripheriegeräte angeschlossen, nämlich eine Eingabeeinheit 24, die beispielsweise eine handelsübliche Computertastatur sein kann, eine erste Ausgabeeinheit 25, die beispielsweise ein üblicher Monitor oder sonstiges Display sein kann, sowie ggf. eine zweite Ausgabeeinheit 26, die ein Drucker sein kann.

Schließlich kann noch eine weitere Eingabeeinheit 27 an die Steuereinheit angeschlossen sein, die zur Erkennung bzw. Identifizierung eines Endeskopes dient. Es kann sich hierbei um ein Lesegerät handeln, das eine Magnetcodierung, einen Strichcode, einen Transponder-Code oder sonstige Identifizierungskennzeichen eines Endoskopes liest und der Steuereinheit meldet, worüber jedes Endoskop individuell identifizierbar ist. Dieses Gerät 27 kann sich auf dem Träger 20 befinden und den Code des jeweiligen Endoskops automatisch lesen. Es kann sich aber beispielsweise auch um ein außerhalb des Trägers 20 angeordnetes Lesegerät handeln, mittels dessen der Benutzer den Code des Endoskopes beim Einlegen in den Träger 20 liest. Selbstverständlich kann die Identifizierung des individuellen Endoskopes auch visuell vorgenommen werden und über die Eingabeinheit 24, also beispielsweise eine Tastatur, eingegeben werden.

Nach Einlegen des Endoskopes auf den Träger 20 und Anschließen der Endoskopkanäle 9 an die Leitungen 8 wird der Träger 20 in eine nicht dargestellte Reinigungs- und Desinfektionsmaschine eingeschoben und über die Kupplungen 17, 18 und 19 mit den sich außerhalb des Waschraumes befindlichen Komponenten verbunden. In den Behältern 1 befindet sich eine Reinigungs- und Desinfektionsflüssigkeit, mit der die Endoskopkanäle gereinigt bzw. desinfiziert werden. Üblicherweise folgt auf einen oder mehrere Reinigungsschritte ein Spüldurchgang mit reinem Wasser. Hierzu werden die Behälter 1 über die Wasserversorgung 4 vollständig oder bis zu einem vorbestimmten Pegel befüllt, so daß sie jeweils ihr vorbestimmtes Flüssigkeitsvolumen enthalten. Mit Hilfe der Druckluftquelle 12 wird dieses vorbestimmte Volumen dann unter vorbestimmtem Druck durch die Endoskopkanäle 9 gedrückt und die Zeitdauer gemessen, bis das Volumen vollständig durch die Kanäle 9 gedrückt ist. Die hierdurch erhaltenen Meßwerte (in ml/s oder bei bekanntem Volumen auch nur in s) werden in der Steuereinheit 16 gespeichert und dabei jedem individuellen Endoskop und dort wiederum jedem individuellen Kanal zugeordnet. Aus diesen Meßwerten erfolgt dann die Trendermittlung, die nachfolgend beschrieben wird.

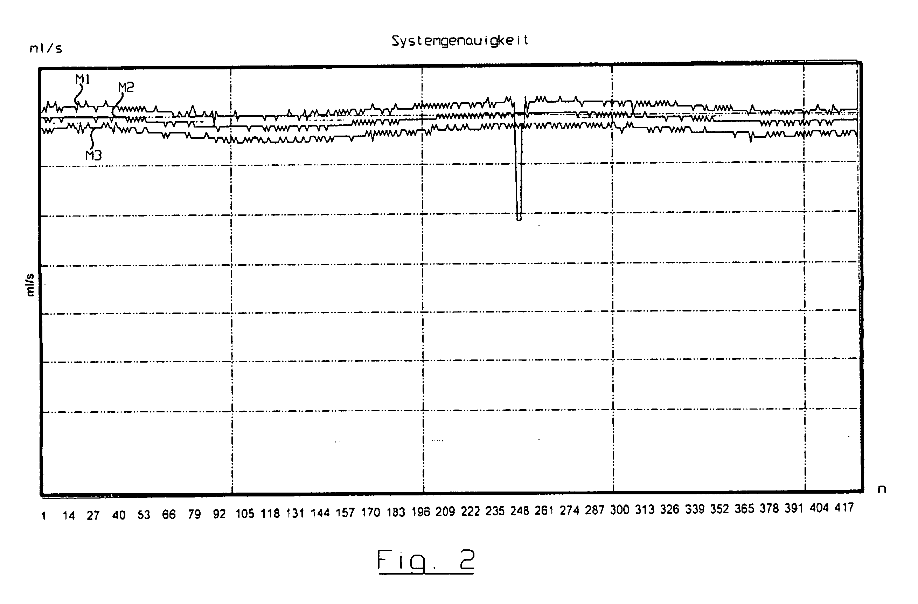

Fig. 2 zeigt drei Meßreihen M1 bis M3, die mit der Vorrichtung der Fig. 1 an einem neuen einwandfreien Endoskop durchgeführt wurden, um die Zuverlässigkeit des Systems selbst zu testen und eventuelle systembedingte Abweichungen zu ermitteln. Als Flüssigkeit wurde reines Wasser verwendet. Die Flüssigkeitsmenge lag zwischen 50 und 1000 ml. Der Druck wurde konstant bei 300 mbar gehalten. Die Meßdauer lag zwischen ein und zwei Minuten. An drei Kanälen wurden 420 Messungen durchgeführt. Die Durchflußrate (Volumen pro Zeiteinheit) in ml/s wurde über die Anzahl n der einzelnen Messungen aufgetragen, was die Meßreihen M1 bis M3 ergab.

Eine Auswertung dieser Meßreihen ergibt, daß die systembedingten Abweichungen in jedem Fall kleiner +/-2% sind. Der starke Einbruch der Meßreihe M1 bei den Messungen 240f ist eine Störung durch einen kurzzeitig blockierten Kanal und daher unbeachtlich. Mit den Meßreihen der Fig. 2 ist somit nachgewiesen, daß der Meßaufbau robust ist und die Meßergebnisse reproduzierbar sind.

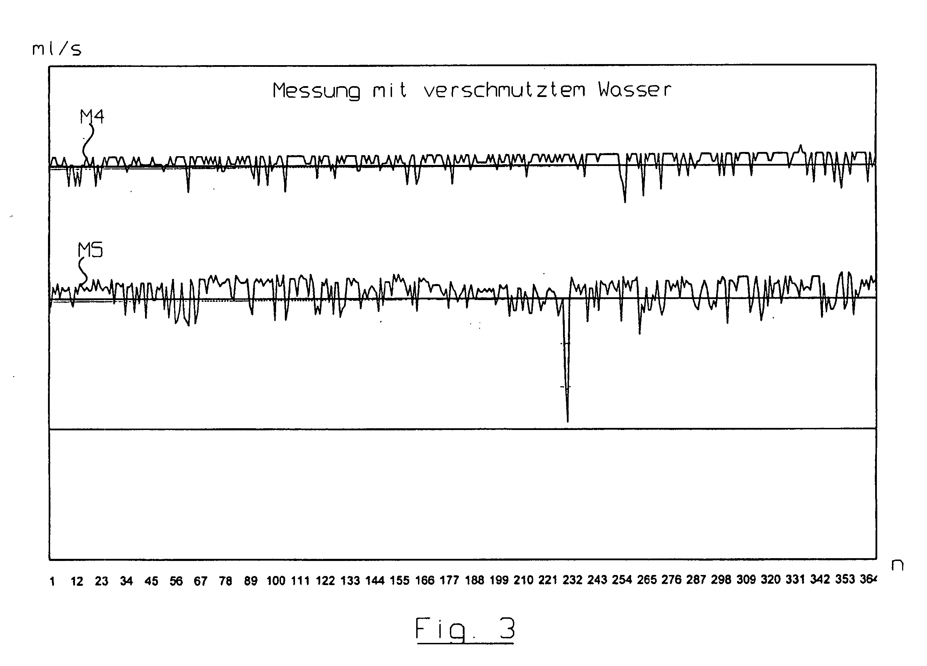

In Fig. 3 sind zwei Meßreihen M4 und M5 über ca. 370 Messungen dargestellt, die mit durch Partikel und organischem Material verschmutztem Wasser durchgeführt wurden. An diesen Meßreihen ist deutlich zu erkennen, daß die Durchflußmenge pro Zeiteinheit einen Maximalwert nur selten überschreitet, von diesem aber sehr häufig nach unten abweicht. Die Reduzierung der Durchflußrate für die einzelnen Messungen in diesen Kanälen ist auf teilweise kontinuierliche oder auf eine kurzzeitige totale Blockierung des Kanals zurückzuführen. Diesen Meßreihen läßt sich aber auch entnehmen, daß aufgetretene Kanalverengungen oder -verstopfungen immer wieder vollständig ausgespült werden, da die Durchflußrate nach ein- oder mehrmaligen Einbrüchen immer wieder auf den Maximalwert zurückkommt.

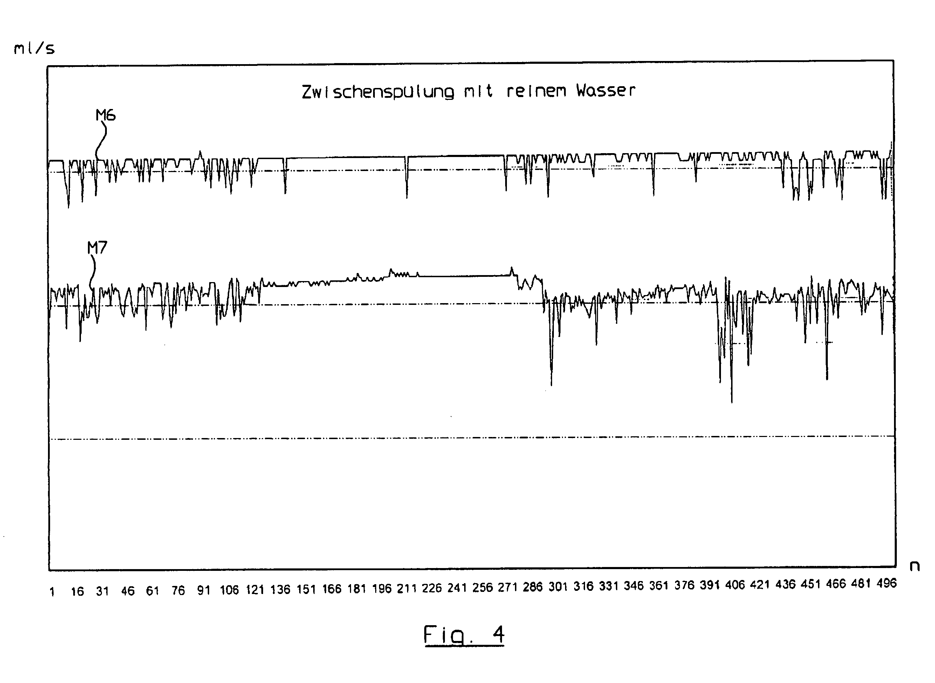

Zur weiteren Überprüfung wurde entsprechend den Meßreihen M6 und M7 in Fig. 4 zunächst ebenfalls mit Partikeln und organischem Material kontaminiertem Wasser gespült und zwischenzeitlich ab ca. der 125. Messung für 150 weitere Messungen mit reinem Wasser gespült und anschließend wieder mit kontaminiertem Wasser. Aus beiden Meßreihen M6 und M7 zeigt sich deutlich, daß das System nach einigen Spülungen mit reinem Wasser wieder in den stabilen Zustand zurückkehrt, bei dem die Durchflußraten im wesentlichen nur noch durch systembedingte Toleranzen variieren. Sobald wieder (ca. Messung 270) auf kontaminiertes Wasser umgeschaltet wurde, zeigten sich vor allem in der Meßkurve M7 wiederum deutliche Abweichungen in Richtung geringerer Durchflußraten.

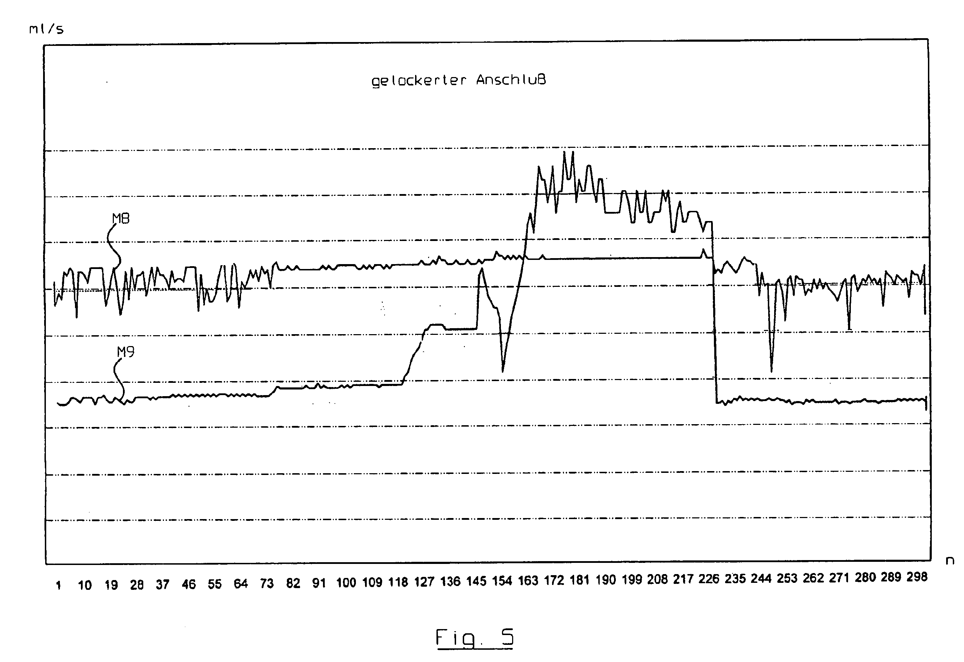

Beim Umbau des Versuchsaufbaus von kontaminiertem Wasser auf nicht kontaminiertes Wasser wurde zufällig die Ankopplung an einen Kanal gelockert. Fig. 5 zeigt im Prinzip dieselben Meßreihen wie Fig. 4, jedoch ist in der Meßreihe M9 der zugeordnete Kanalanschluß gelockert, so daß nicht die vollständige Flüssigkeitsmenge durch den Kanal gedrückt wurde, sondern daneben abfloß, was die Durchflußrate sprunghaft ansteigen ließ. Nachdem der Anschluß bei der Messung 230 wieder korrekt befestigt wurde, ging die Durchflußrate auf den ursprünglichen Wert zurück. Diese Eigenschaft des Systems kann in einfacher Weise dafür benutzt werden, um nicht korrekt angeschlossene Kanäle zu identifizieren und den Reinigungs- bzw. Desinfektionsprozeß abzubrechen und nach Beseitigung des Fehlers neu zu starten.

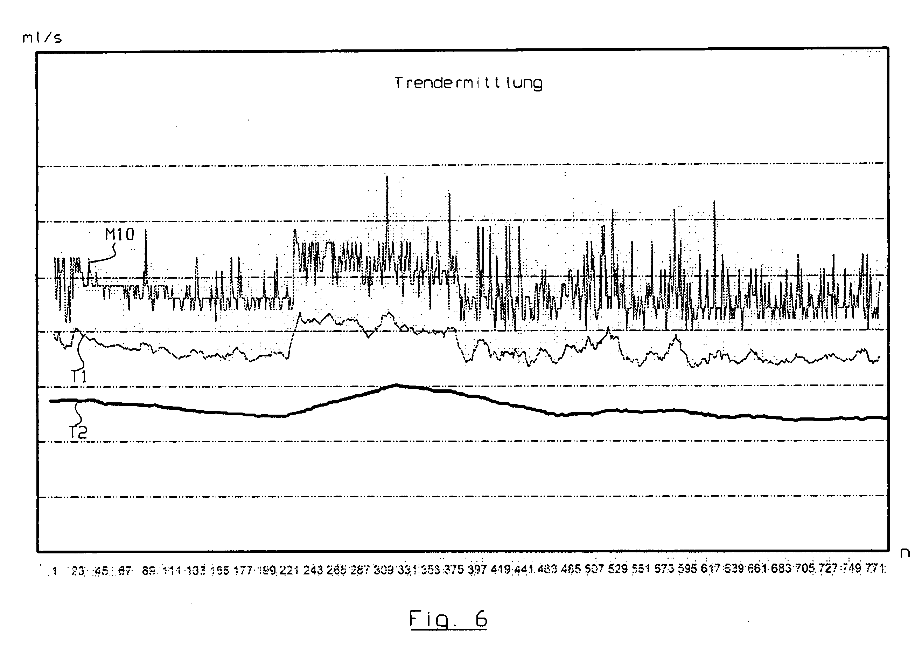

Fig. 6 zeigt eine weitere Meßreihe M10 mit kontaminiertem Wasser über ca. 780 Messungen an einem Kanal. Bei der Messung 220 wurde der gemessene Kanal durch einen absolut gleichartigen, jedoch neuen Kanal ersetzt, was den sprunghaften Anstieg der Meßreihe M10 auf die Durchflußrate eines neuen Kanales erklärt. Bei den weiteren ca. 550 Messungen nimmt die Durchflußrate mit der Zeit wieder ab. Nach ca. 375 Messungen stand das System für eine Nacht still. Bei Wiederaufnahme der Messungen zeigt sich eine deutliche Reduzierung der Durchflußrate, was durch die oben erklärten Phänomene begründet ist.

Sodann wurde anhand der Meßreihe M10 eine Trendanalyse durchgeführt. Die Kurve T1 zeigt eine Trendanalyse nach der Methode des gleitenden Durchschnittes jeweils über die letzten zehn Meßwerte und die Kurve T2 über die jeweils letzten hundert Meßwerte. Es ist offensichtlich, daß die Trendlinie umso "glatter" wird, je mehr zurückliegende Werte in die Mittelwertbildung einbezogen werden und umgekehrt. Bei der Trendlinie T1 wirken sich einzelne Meßwerte noch relativ stark aus, während bei der Trendlinie T2 der langfristige Trend sehr deutlich erkennbar ist.

Allgemein kann die Trendermittlung nach bekannten mathematischen Trendberechnungsmethoden durchgeführt werden. Solche Methoden sind beispielsweise:
Methode der kleinsten Quadrate mit
- linearem: y = mx + b
- polynomischem: y = b + c₁ · x + c₂ · x² + c₃ · x³ + ...
- logarithmischem: y = c lnx + b
- exponentiellem: y = ce^{bx}
Kurvenverlauf
einfacher Mittelwertbildung über alle vorausgegangenen Meßwerte oder gleitendem Durchschnitt mit Mittelwertbildung über nur eine vorgegebene Anzahl vorausgegangener Meßwerte, wobei in Verfeinerung dieser Methode die jüngeren Meßwerte stärker gewichtet werden können als die älteren Meßwerte.

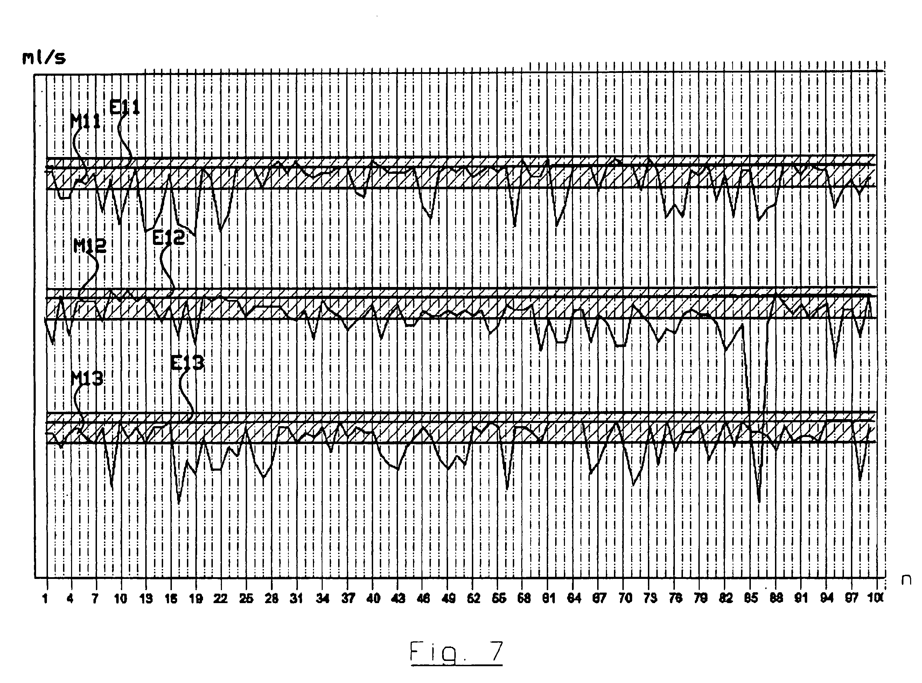

Fig. 7 zeigt noch einmal drei reale Meßkurven M11, M12 und M13 in vergrößertem Maßstab, bei denen die Endoskopkanäle unmittelbar im Anschluß an eine Reinigung und Desinfektion auf ihre Durchlässigkeit gemessen wurden. Aus diesen Kurven wird deutlich, daß es durchaus in der Praxis vorkommt, daß ein Kanal mit einem einzigen oder auch mehreren Spülvorgängen nicht vollständig von einer eingebrachten Verschmutzung gereinigt werden kann. In mehreren Bereichen sind offensichtlich mehrere Spülvorgänge erforderlich, um die Durchflußrate für diesen Kanal wieder auf einen stabilen Maximalwert oder wenigstens nahe dorthin zu bringen. Am auffälligsten ist dies bei der Meßreihe M12, wo die Durchflußrate bei der Messung Nr. 85 und 86 extrem abfällt, bei den Messungen Nr. 87 und 88 wieder steil ansteigt und erst bei Messung Nr. 89 wieder den Maximalwert erreicht.

Dies hat für das erfindungsgemäße Verfahren folgende Konsequenz:
Ein Endoskopkanal wird in einem ersten Arbeitsschritt normal gereinigt und desinfiziert, was auch mehrere Einzelspülungen der Kanäle umfassen kann. Sodann wird mit reinem Wasser die Durchflußrate gemessen. Liegt der Meßwert innerhalb eines Toleranzbandes von beispielsweise 5% um einen Referenzwert, so gilt die Reinigung als ordnungsgemäß und der gemessene Wert wird gespeichert. Liegt der Meßwert dagegen außerhalb des Toleranzbandes, so wird der Meßwert nicht gespeichert und die beiden genannten Schritte der Reinigung und Messung werden so oft wiederholt, bis der Meßwert wieder innerhalb des Toleranzbandes liegt. Liegt ein Meßwert innerhalb des Toleranzbandes nach einer vorgegebenen Anzahl, beispielsweise fünf oder zehn Reinigungen, immer noch nicht in dem Toleranzband, so wird der Vorgang abgebrochen und das Endoskop verworfen. Jeweils nur aus den im Toleranzband liegenden Meßwerten wird dann nach der oben beschriebenen Vorgehensweise die Trendanalyse durchgeführt. Die Trendanalyse bezieht sich also immer nur auf einwandfrei gereinigte Kanäle. Mit ihr läßt sich dann die verbleibende Lebenserwartung des Endoskopes vorausberechnen.

Das Toleranzband ist in Fig. 7 jeweils schraffiert dargestellt. Da Abweichungen nach "oben" zu höheren Durchflußraten gemäß der Erkenntis der Fig. 2 praktisch auszuschließen sind, liegt das Toleranzband "unsymmetrisch" zu einem Erwartungswert E und beträgt beispielsweise nur 2% Abweichung nach oben, aber 5% Abweichung nach unten. Der Erwartungswert wird zunächst an einem neuen Endoskop ermittelt, beispielsweise als Mittelwert von zehn Messungen. Er sei als erster Sollwert bezeichnet. Da sich die Durchflußeigenschaften des Kanales mit der Zeit verschlechtern, wie im Zusammenhang mit Fig. 6 erläutert, kann für spätere Messungen der Erwartungswert durch die Trendlinie festgelegt werden und das Toleranzband um die Trendlinie herum festgelegt werden mit der Maßgabe, daß die Trendlinie selbst innerhalb eines Toleranzbandes um den ersten Sollwert liegt. Nähert sich die Trendlinie diesem Toleranzband um den ersten Sollwert, so läßt sich aus der Trendlinie extrapolieren, wieviele Einsätze mit anschließender Reinigung (und Messung) noch zu erwarten sind, bevor eine Grundüberholung oder ein Austausch erforderlich sind.

Zusammenfassend ist es mit der Erfindung möglich, mit vertretbarem technischen Aufwand Endoskopkanäle und andere enge lange Lumen bezüglich der Veränderung ihrer Durchflußeigenschaften präzise zu analysieren. Veränderungen der Durchflußraten durch die Kanäle, die außerhalb eines systembedingten Toleranzbandes liegen, können sicher erkannt werden. Dies ermöglicht nicht nur die Erkennung von Kanalblockaden sondern auch das frühzeitige Erkennen von Kanalveränderungen, die beispielsweise durch aufwachsenden Biofilm, mechanische Beschädigungen der Kanalinnenwände oder ähnliches verursacht sind. Andererseits lassen sich auch nicht korrekt mit dem System verbundene Kanäle erkennen. Da die Erfindung praktisch gleichzeitig mit der Reinigung und Desinfektion der Kanäle durchgeführt wird, nimmt die Kanalanalyse keine zusätzliche Zeit in Anspruch.

## Patentansprüche

1. Verfahren zur Analyse von Kanälen, insbesondere von Endoskopkanälen mit folgenden Schritten:
- Leiten einer Flüssigkeit durch den zu überprüfenden Kanal,
- Messen eines mit der Durchlässigkeit des Kanales verknüpften Meßwertes,
**gekennzeichnet durch** folgende Schritte:
- Identifizieren des Kanales;
- Speichern des Meßwertes in Zuordnung zu dem identifizierten Kanal;
- mehrfaches Wiederholen der obigen Schritte mit Speichern der Meßwerte in Zuordnung zur Reihenfolge der Messungen und
- Durchführen einer Trendanalyse der Meßwerte nur von einwandfrei gereinigten Kanälen nach an sich bekannten mathematischen Methoden.

2. Verfahren nach Anspruch 1, **dadurch gekennzeichnet, daß** das Verfahren in unmittelbarem Zusammenhang mit der Reinigung und/oder Desinfektion des Kanals durchgeführt wird.

3. Verfahren nach Anspruch 2, **dadurch gekennzeichnet, daß** das Verfahren vor, während oder nach der Reinigung und/oder Desinfektion des Kanals durchgeführt wird.

4. Verfahren nach Anspruch 3, **dadurch gekennzeichnet, daß** die Flüssigkeit mit einem vorbestimmten Druck aus einem Behälter mit einem vorbestimmten Volumen durch den Kanal gedrückt wird und gleichzeitig diejenige Flüssigkeit ist, mit der der Kanal nach dessen Reinigung und/oder Desinfektion gespült wird.

5. Verfahren nach einem der Ansprüche 1 bis 4, **dadurch gekennzeichnet,**
**daß** die Trendanalyse eine Mittelwertbildung der Meßwerte ist.

6. Verfahren nach Anspruch 5, **dadurch gekennzeichnet,**
**daß** die Trendanalyse die Bildung eines sog. gleitenden Mittelwertes ist, bei der der Mittelwert nur aus einer vorgegebenen Anzahl vorangehender Messungen gebildet wird.

7. Verfahren nach einem oder mehreren der Ansprüche 2 bis 6, **dadurch gekennzeichnet,**
**daß** das Reinigen des Kanales mit anschließender Messung so oft durchgeführt wird, bis der Meßwert innerhalb eines vorgegebenen Wertebereiches liegt.

8. Verfahren nach Anspruch 7, **dadurch gekennzeichnet,**
**daß** nur solche Meßwerte gespeichert und zur Trendanalyse verwendet werden, die innerhalb des vorgegebenen Wertebereiches liegen.

9. Verfahren nach Anspruch 7 oder 8, **dadurch gekennzeichnet,**
**daß** der vorgegebene Wertebereich um vorgegebene Prozentwerte um einen ersten vorgegebenen Sollwert liegen, der einem Referenzwert eines einwandfreien Kanales entspricht.

10. Verfahren nach einem der Ansprüche 7 oder 8, **dadurch gekennzeichnet,**
**daß** der vorgegebene Wertebereich um vorgegebene Prozentwerte einer bei der Trendanalyse ermittelten Trendlinie entspricht.

11. Verfahren nach einem der Ansprüche 1 bis 10, **dadurch gekennzeichnet,**
**daß** eine Warnmeldung für eine präventive Wartung ausgegeben wird, wenn die Trendanalyse ergibt, daß die dabei ermittelte Trendlinie einen vorgegebenen Grenzwert unterschreitet.

12. Vorrichtung zur Durchführung des Verfahrens nach einem der Ansprüche 1 bis 11, mit
einer Einrichtung zum Durchleiten einer Flüssigkeit durch einen zu überprüfenden Kanal und einer Meßeinrichtung zum Messen eines auf die Durchlässigkeit des Kanals bezogenen Meßwertes,
**dadurch gekennzeichnet,**
**daß** die Vorrichtung eine Eingabeeinheit (27) aufweist, zur Identifikation des zu überprüfenden Kanals (9),
**daß** die Meßeinrichtung (16) einen Speicher (16") aufweist, in welchem den Meßwerten entsprechende Daten in Zuordnung zu dem identifizierten Kanal (9) und in Zuordnung zur zeitlichen Reihenfolge des Auftretens der Meßwerte speicherbar sind und daß die Meßeinrichtung (16) eine Auswerteeinrichtung (16') aufweist, die eingerichtet ist, nur an einwandfrei gereinigten Kanälen nach einem an sich bekannten mathematischen Verfahren eine Trendanalyse der Meßgrößen durchführt.

## Claims

1. Method for analysis of channels, in particular of endoscope channels with the following steps:
- delivering a fluid through the channel to be checked,
- measuring of a measuring value related to the permeability of the channel,
**characterised by** the following steps:
- identifying the channel;
- storing the measuring value in assignment to the identified channel;
- repeating several times of the above steps with storing of the measuring values in assignment to the order of the measurings and
- performing a trend analysis of the measuring values only of faultlessly cleaned channels according to mathematical methods known per se.

2. Method according to claim 1, **characterised in that** the method is performed in direct relation to the cleaning and/or disinfection of the channel.

3. Method according to claim 2, **characterised in that** the method is performed before, during or after the cleaning and/or disinfection of the channel.

4. Method according to claim 3, **characterised in that** the fluid is pressed through the channel with a predetermined pressure from a container having a predetermined volume and is simultaneously that fluid with which the channel is flushed after its cleaning and/or disinfection.

5. Method according to one of claims 1 to 4, **characterised in that** the trend analysis is an averaging of the measuring values.

6. Method according to claim 5, **characterised in that** the trend analysis is the generating of a so-called gliding average value wherein the average value is made only from a predetermined number of preceding measurings.

7. Method according to one ore more of claims 2 to 6, **characterised in that** the cleaning of the channel with following measuring is performed as often as the measuring value lies within a predetermined measuring range.

8. Method according to claim 7, **characterised in that** only such measuring values are stored and used for the trend analysis, which lie within the predetermined value range.

9. Method according to claim 7 or 8, **characterised in that** the predetermined value range lies by predetermined percentage quotation around a first predetermined set value which corresponds to a reference value of a correct channel.

10. Method according to one of claims 7 or 8, **characterised in that** the predetermined value range corresponds by a predetermined percentage quotation to a trend line determined by the trend analysis.

11. Method according to one of claims 1 to 10, **characterised in that** a warning message is emitted if the trend analysis shows that the trend line determined therewith falls below a predetermined limit value.

12. Device for performing the method according to one of claims 1 to 11 having
means for conveying a fluid through a channel to be checked and with a measuring device for measuring of a measuring value referred to the permeability of the channel, **characterised in that**
the device comprises an input unit (27) for identification of the channel (9) to be checked, **in that** the measuring device (16) comprises a memory (16"), wherein data related to the measuring values can be stored in assignment to the identified channel (9) and in assignment to the temporary order of the appearance of the measuring values and
**in that** the measuring device (16) comprises an evaluation device (16') adapted to perform a trend analysis of the measuring values according to a mathematical method which is known per se only with respect to correctly cleaned channels.

## Revendications

1. Procédé pour analyser des canaux, en particulier des canaux d'endoscope, comprenant les étapes suivantes :
- acheminement d'un fluide dans le canal à contrôler,
- réalisation d'une mesure associée à la conductance du canal,
**caractérisé par** les étapes suivantes :
- identification du canal ;
- enregistrement de la mesure en association avec le canal identifié ;
- répétitions multiples des étapes ci-dessus avec enregistrement des mesures en association avec l'ordre des mesures et
- en utilisant des méthodes mathématiques connues, réalisation d'une analyse de tendance des mesures uniquement des canaux nettoyés parfaitement.

2. Procédé selon la revendication 1, **caractérisé en ce que** le procédé est réalisé en relation directe avec le nettoyage et/ou la désinfection du canal.

3. Procédé selon la revendication 2, **caractérisé en ce que** le procédé est réalisé avant, pendant ou après le nettoyage et/ou la désinfection du canal.

4. Procédé selon la revendication 3, **caractérisé en ce que** le fluide est injecté sous pression dans le canal à une pression prédéterminée à partir d'un conteneur présentant un volume prédéterminé et en même temps c'est avec ce même fluide que le canal est rincé après son nettoyage et/ou sa désinfection.

5. Procédé selon l'une des revendications 1 à 4, **caractérisé en ce que** l'analyse de tendance est la formation d'une moyenne de mesures.

6. Procédé selon la revendication 5, **caractérisé en ce que** l'analyse de tendance est la formation d'une moyenne dite glissante, dans laquelle la moyenne est formée uniquement à partir d'un nombre prédéterminé de mesures existantes.

7. Procédé selon une ou plusieurs des revendications 2 à 6, **caractérisé en ce que** le nettoyage du canal avec mesure consécutive est réalisé jusqu'à ce que la mesure se situe dans une plage de valeurs prédéterminées.

8. Procédé selon la revendication 7, **caractérisé en ce que** seules sont enregistrées, et utilisées pour l'analyse de tendance, les mesures qui se situent dans la plage de valeurs prédéterminées.

9. Procédé selon la revendication 7 ou 8, **caractérisé en ce que** la plage de valeurs prédéterminées autour des pourcentages prédéterminés se situe autour d'une première valeur théorique prédéterminée qui correspond à une valeur de référence d'un canal en parfait état.

10. Procédé selon l'une des revendications 7 ou 8, **caractérisé en ce que** la plage de valeurs prédéterminées autour de pourcentages prédéterminés correspond à une ligne de tendance déterminée lors de l'analyse de tendance.

11. Procédé selon l'une des revendications 1 à 10, **caractérisé en ce qu'**un message d'avertissement est émis pour un entretien préventif lorsque l'analyse de tendance indique que la ligne de tendance ainsi déterminée dépasse une valeur seuil prédéterminée.

12. Dispositif pour la mise en oeuvre du procédé selon l'une des revendications 1 à 11, comportant
un appareil pour injecter un fluide dans un canal à contrôler et un appareil de mesure pour déterminer une mesure relative à la conductance du canal,
**caractérisé**
**en ce que** le dispositif comprend une unité d'entrée (27) pour l'identification du canal à contrôler (9),
**en ce que** l'appareil de mesure (16) comprend une mémoire (16") dans laquelle les données correspondant aux mesures peuvent être enregistrées en association avec le canal identifié (9) et en association avec l'ordre temporel de survenue des mesures et
**en ce que** l'appareil de mesure (16) comprend un dispositif d'analyse (16'), lequel est mis en oeuvre pour réaliser, uniquement sur des canaux parfaitement nettoyés, selon une méthode mathématique connue, une analyse de tendance des mesures.
